(19) Europäisches Patentamt
European Patent Office
Office européen des brevets

(11) Veröffentlichungsnummer: **0 003 767**
**B1**

(12) # EUROPÄISCHE PATENTSCHRIFT

(45) Veröffentlichungstag der Patentschrift: **21.10.81**

(21) Anmeldenummer: **79100341.1**

(22) Anmeldetag: **06.02.79**

(51) Int. Cl.³: **C 07 C 143/675,**
C 07 D 295/08,
C 09 B 29/30

(54) **N-Acylaminohydroxynaphthalinsulfonsäuresalze, Verfahren zu ihrer Herstellung und ihre Verwendung als Kupplungskomponenten zur Herstellung von Azofarbstoffen.**

(30) Priorität: **18.02.78 DE 2806951**

(43) Veröffentlichungstag der Anmeldung:
**05.09.79 Patentblatt 79/18**

(45) Bekanntmachung des Hinweises auf die Patenterteilung:
**21.10.81 Patentblatt 81/42**

(84) *Benannte Vertragsstaaten:*
**CH DE FR GB**

(56) Entgegenhaltungen:
**DE - A - 1 810 279**
**US - A - 2 698 342**

(73) Patentinhaber: **BAYER AG**
**Zentralbereich Patente, Marken und Lizenzen**
**D-5090 Leverkusen 1, Bayerwerk (DE)**

(72) Erfinder: **Wild, Peter, Dr.**
**Hainstrasse 7**
**D-6305 Alten Buseck (DE)**
Erfinder: **Nickel, Horst, Dr.**
**Fontanestrasse 23**
**D-5090 Leverkusen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**EP 0 003 767 B1**

N-Acylaminohydroxynaphthalinsulfonsäuresalze, Verfahren zu ihrer Herstellung und ihre Verwendung als Kupplungskomponenten zur Herstellung von Azofarbstoffen

Die Erfindung betrifft Salze von N-Acylaminohydroxynaphthalinsulfonsäuren, die in Form der freien Säure der Formel

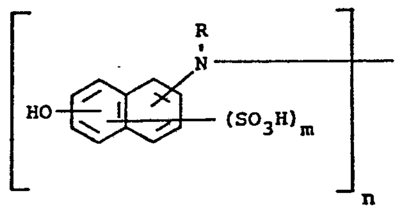

(I)

entsprechen,
worin

R Wasserstoff oder Alkyl,
X den Rest einer ein- oder zweiwertigen Acylgruppe,
m 1 oder 2 und
n 1 oder 2 bedeuten

und worin wenigstens eine der Sulfonsäuregruppen an Stelle des Protons ein Kation der Formel

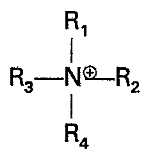

enthält, worin

$R_1$ und $R_2$ unabhängig voneinander $C_1$—$C_4$-Alkyl, $C_1$—$C_4$-Alkoxy-$C_1$—$C_4$-alkyl oder $R_3$ oder $R_1$ und $R_2$ zusammen mit dem Stickstoffatom Piperidinyl, Morpholinyl oder Pyrrolinyl,

$R_3$ die Gruppe

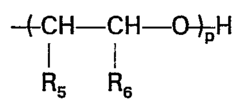

$R_4$ Wasserstoff, $C_1$—$C_4$-Alkyl, $C_1$—$C_4$-Alkoxy-$C_1$—$C_4$-alkyl oder die Gruppe

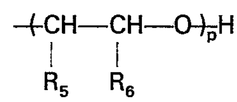

und

$R_5$ und $R_6$ Wasserstoff, Methyl oder Äthyl bedeuten und
p für eine ganze Zahl 1 bis 10 steht.

Falls n 2 bedeutet, kann der Farbstoff aus gleichen oder ungleichen Aminohydroxynaphthalinsulfonsäuren aufgebaut sein.

Die neuen Verbindungen der Formel (I) werden dadurch erhalten, daß man die freien Säuren mit entsprechenden Acylierungsmitteln in Gegenwart von Verbindungen der Formel

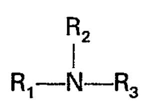

oder ihrer durch $C_1$—$C_4$-Alkyl, $C_1$—$C_4$-Alkoxy-$C_1$—$C_4$-alkyl oder Gruppen der Formel

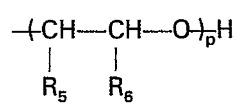

quaternierten Hydroxyde,
worin

$R_1$, $R_2$, $R_3$, $R_5$, $R_6$ und p die o.g. Bedeutungen haben, umsetzt.

Geeignete Alkylgruppen R sind insbesondere $C_1$—$C_4$-Alkyl, vorzugsweise Methyl oder Äthyl.

Geeignete einwertige Acylgruppen sind $C_1$—$C_4$-Alkylcarbonyl und gegebenenfalls durch Methyl, Methoxy, Chlor oder Nitro substituiertes Benzoyl. Geeignete zweiwertige Acylgruppen sind —CO—CO—, —CO—, —CO—CH=CH—CO und Terephthaloyl.

Als Acylierungsmittel kommen die Halogenide und Anhydride von aliphatischen und aromatischen Carbonsäuren, deren Acylrest der obigen Definition entspricht, in Frage, sowie Kohlensäurederivate wie Phosgen oder Chlorameisensäuremethyl-, äthyl- oder -phenylester und Oxalsäure-, Fumarsäure- und Terephthalsäuredichlorid.

Geeignete Aminohydroxynaphthalinsulfonsäuren sind beispielsweise 7-Amino-4-hydroxy-2-naphthalinsulfonsäure, 8-Amino-4-hydroxy-2-naphthalinsulfonsäure, 7-Amino-3-hydroxy-1-naphthalinsulfonsäure, 7-Methylamino-4-hydroxy-2-naphthalinsulfonsäure, 6-Amino-4-hydroxy-2-naphthalinsulfonsäure, 6-Amino-4-hydroxy-1-naphthalinsulfonsäure, 2-Amino-5-hydroxy-1,7-naphthalindisulfonsäure, 3-Amino-8-hydroxy-1,5-naphthalindisulfonsäure und 3-Amino-5-hydroxy-2,7-naphthalindisulfonsäure.

Die Acylierung wird zweckmäßigerweise so durchgeführt, daß man die Aminohydroxysulfonsäuren als freie Säuren in Wasser löst oder suspendiert, evtl. unter Zusatz eines polaren organischen Lösungsmittels wie Dimethylformamid, Sulfolan oder Glykoläthern mit dem tertiären Amin oder seinem quaternierten Hydroxid neutral stellt und das Acylierungsmittel zugibt. Die bei der Reaktion freiwerdende Säure wird durch Zugabe des gleichen oder eines anderen tertiären Amins oder dessen quatären Hydroxids neutralisiert und der pH-Wert zwischen etwa 5 und 8 gehalten. Man führt die Reaktion zweckmäßigerweise bei Temperaturen zwischen 20 und 80°C durch. Weiter ist es zweckmäßig, die Wasser- bzw. Lösungsmittelmenge so zu bemessen, daß wenigstens das Endprodukt in konzentrierter, homogener Lösung vorliegt.

Geeignete Amine und quaternierte Ammoniumhydroxide sind beispielsweise Triäthanolamin, Methyldiäthanolamin, Tris-Propanol- oder -isopropanolamin, Tris-[2-(2-Hydroxyäthoxy)äthyl]amin, Diäthyl- oder Dimethyläthanolamin, N-Hydroxyäthylpiperidin, N-Hydroxyäthylmorpholin und die in US—PS3 995 997 genannten quartären Ammoniumhydroxide.

Die erfindungsgemäßen Verbindungen eignen sich sowohl in ihrer isolierten Form als auch insbesondere in Form konzentrierter wäßriger oder wäßrig-organischer Lösungen als Kupplungskomponenten zur Herstellung von Azofarbstoffen, insbesondere zur Herstellung von konzentrierten wäßrigen oder wäßrig-organischen Lösungen von Azofarbstoffen.

Die konzentierten Lösungen der erfindungsgemäßen Verbindungen sind über lange Zeit lagerstabil. Aus diesen Lösungen lassen sich die erfindungsgemäßen Verbindungen durch Einengen oder Abkühlen in fester oder öliger, sehr leicht wasserlöslicher Form isolieren.

Im Vergleich zu den aus der US—PS 2 698 342 bekannten Salzen aus N-acylierten, sulfonsäuregruppenhaltigen aromatischen Aminen und stickstoffhaltigen organischen Basen sowie den aus der DE—OS 1 810 279 bekannten Natriumsalzen von substituierten 2-Aminonaphthalin-1,5-disulfonsäuren lassen sich die erfindungsgemäßen Verbindungen zu wesentlich höherkonzentrierten Lösungen von Azofarbstoffen umsetzen.

### Beispiel 1

120 g 7-Amino-4-hydroxy-2-naphthalinsulfonsäure (I-Säure) werden in 200 ml Wasser und 50 ml Methyldiäthanolamin bei pH 7 bis 8 gelöst. Innerhalb von 2 Stunden wird bei 30 bis 40°C mit 60 ml Acetanhydrid acyliert und der pH-Wert mit etwa 50 ml Methyldiäthanolamin bei 5 bis 6 gehalten. Man rührt nach bis keine I-Säure mehr nachweisbar ist. Die so erhaltene konzentrierte Acetyl-I-Säure-Lösung kann direkt zu Farbstoffen weiterverarbeitet werden. Durch Abkühlen auf Temperaturen unterhalb 0°C scheidet sich das Methyldiäthanolammoniumsalz der Acetyl-I-Säure ab und kann als hellbraunes Pulver isoliert werden. Destilliert man von der Lösung das Wasser ab, so entsteht ein Öl, das nach einiger Zeit kristallin wird.

### Beispiel 2

120 g I-Säure werden mit 300 ml Wasser und etwa 50 bis 60 ml Methyldiäthanolamin bei pH 7 bis 8 gelöst. Bei 50 bis 60°C wird Phosgen eingeleitet und der pH-Wert mit Methyldiäthanolamin bei 6 bis 6,5 gehalten. Wenn der I-Säuregehalt unter 10% gesunken ist, wird bei pH 7 in etwa einer Stunde zuende phosgeniert. Die so erhaltene I-Säureharnstofflösung ist über längere Zeit stabil. Kühlt man auf Temperaturen unterhalb —5°C ab, so scheidet sich das Ammoniumsalz des I-Säureharnstoffs kristallin ab.

### Beispiel 3

Man verfährt wie in Beispiel 1 beschrieben, verwendet jedoch als Acylierungsmittel ca. 60 ml Benzoylchlorid hält den pH-Wert mit 80 ml Methyldiäthanolamin bei 5 bis 6. Nach beendeter Reaktion

kann aus der Lösung das Ammoniumsalz der Benzoyl-I-Säure bei etwa 0°C und mit wenig verdünnter Salzsäure kristallin abgeschieden werden.

Beispiel 4

159,5 g 3-Amino-8-hydroxy-1,5-naphthalindisulfonsäure werden in 300 ml Wasser und 138 ml Methyldiäthanolamin bei 50°C und pH 6,5 gelöst. In diese Lösung leitet man bei 50°C Phosgen ein und hält den pH-Wert mit Methyldiäthanolamin bei 6,5, bis kein Ausgangsprodukt mehr nachweisbar ist. Von der Lösung wird das Wasser weitgehend abdestilliert. Aus der verbleibenden zähen Masse kristallisiert nach einiger Zeit das Methyldiäthanolammoniumsalz des 8,8′-Dihydroxy-1,1′, 5,5′-tetrasulfo-3,3′-di-naphthylharnstoff aus.

Beispiel 5

120 g 7-Amino-4-hydroxy-2-naphthalinsulfonsäure werden in 200 Wasser und etwa 150 ml Tris-[2-(2-hydroxyäthoxy)-äthyl]-amin bei pH 7 bis 8 gelöst. Bei 50 bis 60°C wird Phosgen eingeleitet und der pH-Wert durch Zugabe des gleichen Amins bei 6 bis 7 gehalten, bis das Ausgangsmaterial vollständig zum entsprechenden Harnstoff umgesetzt ist. Die erhaltene Lösung des Harnstoff ist längere Zeit lagerungsstabil und auch direkt als Kupplungsmedium verwendbar.

Beispiel 6

Man verfährt wie in Beispiel 2 angegebenen, jedoch unter Verwendung von N-Hydroxyäthylmorpholin als Amin. Man erhält ca. 600 ml Lösung des N-Hydroxyäthylmorpholiniumsalzes des I-Säureharnstoffs.

Beispiel 7

120 g 6-Amino-4-hydroxy-1-naphthalinsulfonsäure werden in 200 ml Wasser und 120 ml Methyldiäthanolamin bei pH 8,5 suspendiert. Bei 50°C wird Phosgen eingeleitet und der pH-Wert, nachdem er auf 6.5 gefallen ist, mit Methyldiäthanolamin bei diesem Wert gehalten, bis kein Ausgangsprodukt mehr vorhanden ist. Dabei entsteht eine klare Lösung des 4,4′-Dihydroxy-1,1′-disulfo-6,6′-dinapthylharnstoffs als Methyldiäthanolammoniumsalz.

Beispiel 8

Verwendet man in Beispiel 7 anstelle des Methyldiäthanolamins 130 g Methyltriäthanolammoniumhydroxid, so erhält man ebenfalls eine klare Lösung, wobei 4,4′-Dihydroxy-1,1′-disulfo-6,6′-dinaphthyl-harnstoff als Methyltriäthanolammoniumsalz vorliegt.

**Patentansprüche**

1. Salze von N-Acylaminohydroxynaphthalinsulfonsäure, die in Form der freien Säure der Formel

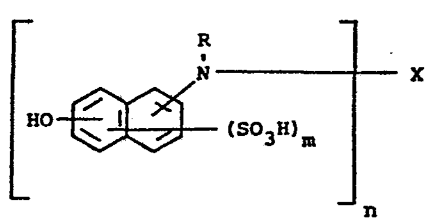

(I)

entsprechen,
worin

R Wasserstoff oder Alkyl,
X den Rest einer ein- oder zweiwertigen Acylgruppe,
m 1 oder 2 und
n 1 oder 2 bedeuten

und worin wenigstens eine der Sulfonsäuregruppen als Kation ein Kation der Formel

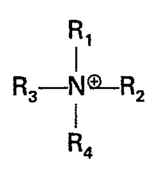

enthält, worin

$R_1$ und $R_2$ unabhängig voneinander $C_1$—$C_4$-Alkyl, $C_1$—$C_4$-Alkoxy-$C_1$—$C_4$-alkyl oder $R_3$ oder $R_1$ und

$R_2$ zusammen mit dem Stickstoffatom Piperidinyl, Morpholinyl oder Pyrrolinyl,

$R_3$ die Gruppe

$$-(\underset{R_5}{CH}-\underset{R_6}{CH}-O)_pH$$

$R_4$ Wasserstoff, $C_1$—$C_4$-Alkyl, $C_1$—$C_4$-Alkoxy-$C_1$—$C_4$-alkyl oder die Gruppe

$$-(\underset{R_5}{CH}-\underset{R_6}{CH}-O)_pH$$

und

$R_5$ und $R_6$ Wasserstoff, Methyl oder Äthyl bedeuten und

p für eine ganze Zahl 1 bis 10 steht.

2. Konzentrierte wäßrige oder wäßrig-organische Lösungen der Verbindungen gemäß Anspruch 1

3. Verfahren zur Herstellung von Verbindungen gemäß Anspruch 1, dadurch gekennzeichnet, daß man Verbindungen der Formel

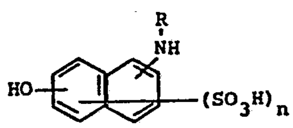

worin R und m die in Anspruch 1 genannte Bedeutung haben in Gegenwart von Aminen der Formel

$$R_1-\overset{R_2}{\overset{|}{N}}-R_3$$

worin $R_1$, $R_2$ und $R_3$ die in Anspruch 1 genannte Bedeutung haben oder ihrer durch $C_1$—$C_4$-Alkyl, $C_1$—$C_4$-Alkoxy-$C_1$—$C_4$-alkyl oder Gruppen der Formel

$$-(\underset{R_5}{CH}-\underset{R_6}{CH}-O)_pH$$

worin $R_5$, $R_6$ und p die in Anspruch 1 angegebene Bedeutung haben, quaternierten Hydroxide unter Einführung der Gruppe X acyliert.

4. Verwendung der Verbindungen gemäß Anspruch 1 als Kupplungskomponenten zur Herstellung von Azofarbstoffen.

5. Verwendung der Verbindungen gemäß Anspruch 1 als Kupplungskomponenten zur Herstellung von konzentrierten Lösungen von Azofarbstoffen.

6. Verwendung der konzentrierten Lösungen gemäß Anspruch 2 zur Herstellung von konzentrierten Lösungen von Azofarbstoffen.

**Revendications**

1. Sels d'acide N-acylaminohydroxynaphtalènesulfonique qui, sous la forme de l'acide libre, répondent à la formule:

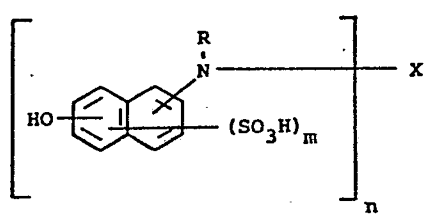

(I)

dans laquelle

R est l'hydrogène ou un groupe alkyle,

X est le reste d'un groupe acyle monovalent ou divalent,

*m* est égal à 1 ou 2 et

*n* est égal à 1 ou 2

et dans laquelle au moins l'un des groupes acide sulfonique renferme comme cation un cation de formule:

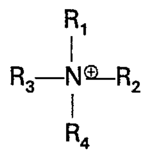

dans laquelle

$R_1$ et $R_2$ représentent indépendamment l'un de l'autre un groupe alkyle en $C_1$ à $C_4$, (alkoxy en $C_1$ à $C_4$)-alkyle en $C_1$ à $C_4$ ou $R_3$, ou bien $R_1$ et $R_2$ forment avec l'atome d'azote un groupe pipéridinyle, morpholinyle ou pyrrolinyle, $R_3$ est le groupe de formule:

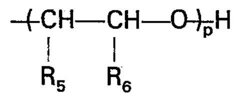

$R_4$ est l'hydrogène, un groupe alkyle en $C_1$ à $C_4$, (alkoxy en $C_1$ à $C_4$)-alkyle en $C_1$ à $C_4$ ou le groupe:

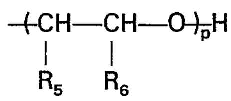

et

$R_5$ et $R_6$ représentent l'hydrogène, un groupe méthyle ou un groupe éthyle et

*p* est un nombre entier de 1 à 10.

2. Solutions aqueuses ou hydro-organiques concentrées des composés suivant la revendiction 1.

3. Procédé de production de composés suivant la revendication 1, caractérisé en ce qu'on acyle des composés de formule:

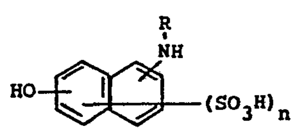

dans laquelle

R et *m* ont la définition donnée dans la revendication 1 en présence d'amines de formule:

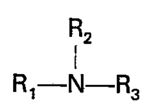

dans laquelle $R_1$, $R_2$ et $R_3$ ont la définition donnée dans la revendication 1

ou de leurs hydroxydes quaternisés par un groupe alkyle en $C_1$ à $C_4$, (alkoxy en $C_1$ à $C_4$)-alkyle en $C_1$ à $C_4$ ou des groupes de formule:

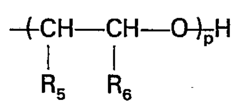

dans laquelle $R_5$, $R_6$ et *p* ont la définition donnée dans la revendication 1, avec introduction du groupe X.

4. Utilisation des composés suivant la revendication 1 comme copulants pour la production de colorants azoïques.

5. Utilisation des composés suivant la revendication 1 comme copulants pour la production de solutions concentrées de colorants azoïques.

6. Utilisation des solutions concentrées suivant la revendication 2 pour la production de solutions concentrées de colorants azoïques.

**Claims**

1. Salts of N-acylaminohydroxynaphthalenesulphonic acid which, in the form of the free acid, correspond to the formula

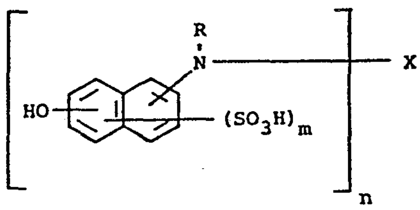

(I)

wherein

R denotes hydrogen or alkyl,

X denotes the radical of a monovalent or divalent acyl group

m denotes 1 or 2 and

n denotes 1 or 2,

and wherein at least one of the sulphonic acid groups contains, as a cation, a cation of the formula

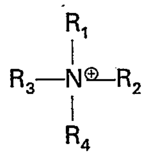

wherein

$R_1$ and $R_2$ independently of one another denote $C_1$—$C_4$-alkyl, $C_1$—$C_4$-alkoxy-$C_1$—$C_4$-alkyl or $R_3$, or $R_1$ and $R_2$, together with the nitrogen atom, denote piperidinyl, morpholinyl or pyrrolinyl,

$R_3$ denotes the group

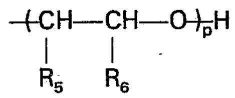

$R_4$ denotes hydrogen, $C_1$—$C_4$-alkyl, $C_1$—$C_4$-alkoxy-$C_1$—$C_4$-alkyl or the group

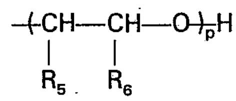

and

$R_5$ and $R_6$ denote hydrogen, methyl or ethyl and

p represents an integer between 1 and 10.

2. Concentrated aqueous or aqueous-organic solutions of the compounds according to Claim 1.

3. Process for the preparation of compounds according to Claim 1, characterised in that compounds of the formula

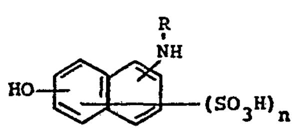

wherein

R and m have the meaning given in Claim 1, are acylated in the presence of amines of the formula

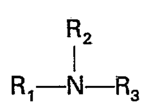

wherein

$R_1$, $R_2$ and $R_3$ have the meaning given in Claim 1, or their hydroxides quaternised by $C_1$—$C_4$-alkyl, $C_1$—$C_4$-alkoxy-$C_1$—$C_4$-alkyl or groups of the formula

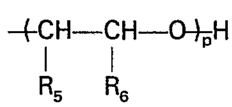

wherein

$R_5$, $R_6$ and p have the meaning given in Claim 1, the group X being introduced.

4. Use of the compounds according to Claim 1 as coupling components for the preparation of azo dyestuffs.

5. Use of the compounds according to Claim 1 as coupling components for the preparation of concentrated solutions of azo dyestuffs.

6. Use of the concentrated solutions according to Claim 2 for the preparation of concentrated solutions of azo dyestuffs.